# EUROPEAN PATENT SPECIFICATION

(11) **EP 1 954 677 B1**
(45) Date of publication and mention of the grant of the patent: **28.07.2010**
(21) Application number: 06797654.8
(22) Date of filing: 01.09.2006
(51) Int. Cl.: C07D 211/58, A61K 31/541, A61P 1/04, A61P 1/06, A61P 1/08, A61P 7/02, A61P 9/00, A61P 9/04, A61P 9/12, A61P 11/00, A61P 11/02, A61P 11/06, A61P 11/10, A61P 11/14, A61P 13/02, A61P 13/10, A61P 17/00, A61P 17/04, A61P 17/06, A61P 19/02, A61P 19/10

(54) **THIOMORPHOLINE COMPOUND AND PROCESS FOR PREPARING THE SAME**
THIOMORPHOLINVERBINDUNG UND VERFAHREN ZU DEREN HERSTELLUNG
COMPOSÉ DE THIOMORPHOLINE ET SON PROCÉDÉ DE PRÉPARATION

(30) Priority: 01.09.2005 JP 2005253105
(43) Date of publication of application: 13.08.2008
(73) Proprietor: Mitsubishi Tanabe Pharma Corporation, Osaka 541-8505 (JP)
(72) Inventor: TAKAHASHI, Masami, Chuo-ku, Osaka-shi, Osaka 541-8505 (JP); YAMANAKA, Takeshi, Chuo-ku, Osaka-shi, Osaka 541-8505 (JP); MIYAKE, Tsutomu, Chuo-ku, Osaka-shi, Osaka 541-8505 (JP)
(74) Representative: HOFFMANN EITLE
(86) International application number: PCT/JP2006/317798
(87) International publication number: WO 2007/026966

(56) References cited:
- WO-A-03/099787
- JP-A- 2004 143 139
- US-A- 4 263 433
- CHEN J.J. ET AL.: 'Catalyzed double Michael addition of anilines to vinyl sulfone' TETRAHEDRON LETTERS vol. 44, no. 17, 2003, pages 3459 - 3462, XP004417833

## Description

### [Technical field]

The present invention relates to a novel thiomorpholine compound having excellent tachykinin receptor antagonistic activity, and preparing the piperidine compound.

### [Background art]

Tachykinin is a general name for a group of neuropeptides, and there have been known substance P (hereinafter referred to as "SP"), neurokinin-A, and neurokinin-B in mammals. These peptides are known to exhibit various kinds of biological activities by binding their corresponding receptors which exist in vivo (neurokinin-1, neurokinin-2, neurokinin-3). Among them, SP is one of those which have been studied the longest and in detail. Its existence was confirmed in an extract of horse intestinal tube in 1931, and it was a peptide comprising 11 amino acids, whose structure was determined in 1971.

SP exists widely in central and peripheral nervous systems, and it has physiological activities such as vasodilative action, vascular permeability promoting action, smooth muscle contracting action, neuronal excitatory action, salivary action, diuretic action, immunological action, etc., as well as a function of neurotransmitter of the primary sensory neuron. Especially, it is known that SP released from the terminal of posterior horn of spinal cord upon pain impulse transfers pain information to the secondary sensory neuron, and that SP released from the peripheral terminus induces an inflammatory response via its receptors. From these facts, SP is considered to be involved in various diseases (for example, pain, inflammation, allergy, pollakiuria, urinary incontinence, respiratory disease, mental disorder, depression, anxiety, emesis, etc.), and also, SP is considered to be involved in Alzheimer-type dementia [Review: Physiological Reviews, vol.73, pp. 229-308 (1993) , Journal of Autonomic Pharmacology, vol.13, pp. 23-93 (1993) .

Currently, as a compound having tachykinin receptor antagonistic activity, in WO 2003/011860 , there is described a compound represented by the following formula: 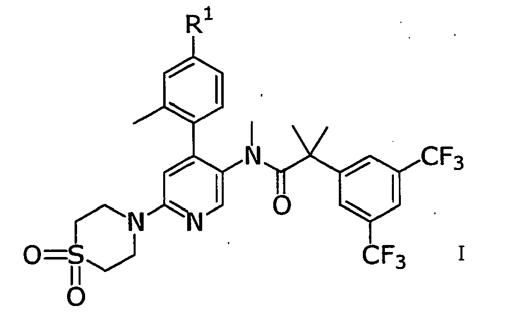 wherein R¹ represents hydrogen or fluoro, and a pharmaceutically acceptable acid addition salt.
Also, in WO 2002/032867, there is described a compound represented by the following formula: 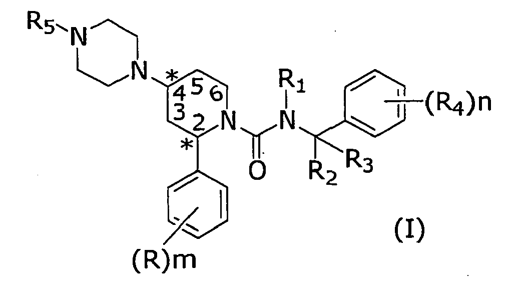 wherein R represents a halogen atom or a C₁₋₄ alkyl group;
R₁ represents a C₁₋₄ alkyl group;
R₂ represents hydrogen or a C₁₋₄ alkyl group;
R₃ represents hydrogen or a C₁₋₄ alkyl group;
R₄ represents trifluoromethyl group;
R₅ represents hydrogen, a C₁₋₄ alkyl group or C (O) R₆;
R₆ represents a C₁₋₄ alkyl, a C₃₋₇ cycloalkyl, a NH(C₁₋₄ alkyl) or a N(C₁₋₄ alkyl)₂ ;
m is 0 or an integer of 1 to 3;
n is an integer of 1 to 3,
or a pharmaceutically acceptable salt or a solvated compound thereof, in WO2003/066589 , there is described a compound represented by the formula: 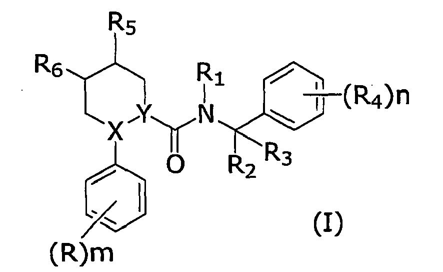 wherein:
R represents a halogen or a C₁₋₄ alkyl;
R₁ represents hydrogen or a C₁₋₄ alkyl;
R₂ represents hydrogen, a C₁₋₄ alkyl, or R₂ and R₃ are combined to form a C₃₋₇ cycloalkyl;
R₃ represents hydrogen, a C₁₋₄ alkyl, a C₃₋₇ cycloalkyl or a C₃₋₆ alkenyl; or R₁ and R₃ form a 5- or 6-membered heterocyclic group with the nitrogen and carbon atoms to which they are bonded in combination;
R₄ represents trifluoromethyl, a C₁₋₄ alkyl, a C₁₋₄ alkoxy, trifluoromethoxy or a halogen;
R₅ is hydrogen, and R₆ is NR₇R₈, or R₅ is NR₈R₉ and R₆ is hydrogen;
R₇ represents hydrogen or a C₁₋₄ alkyl, or R₇ and R₈ form a saturated 5- to 7-membered heterocyclic group containing oxygen with the nitrogen to which they are bonded in combination;
R₈ represents hydrogen, phenyl, a C₃₋₇ cycloalkyl, (CH₂)ₚC(O)NR₁₀R₁₁, a saturated 5- to 7-membered heterocyclic group (which may be substituted by a C₁₋₄ alkyl, a S(O)₂C₁₋₄ alkyl or a C(O)C₁₋₄ alkyl) containing 1 to 3 hetero atoms selected from oxygen, sulfur and nitrogen, a 5-membered heteroaryl group (which may be substituted by a C₁₋₄ alkyl S(O)₂C₁₋₄ alkyl or a C(O)C₁₋₄ alkyl) containing 1 to 3 hetero atoms selected from oxygen, sulfur and nitrogen, or R₈ is a 6-membered heteroaryl group (which may be substituted by a C₁₋₄ alkyl, a S(O)₂C₁₋₄ alkyl or a C(O)C₁₋₄ alkyl) containing 1 to 3 nitrogen atoms; or R₈ is a C₁₋₆ alkyl group which may be optionally substituted by 1 or 2 groups selected from fluorine, phenyl (which may be substituted by a C₁₋₄ alkyl, a C(O)C₁₋₄ alkyl or a halogen), =O, a C₃₋₇ cycloalkyl, hydroxy, amino, dimethylamino, aminocarbonyl, a C₁₋₄ alkoxy and trifluoromethyl;
R₉ represents hydrogen or a C₁₋₄ alkyl, or R₉ and R₈ may form a 5- to 7-membered heterocyclic group with the nitrogens to which they are bonded, which may contain another one hetero atom selected from oxygen, sulfur and nitrogen, and may be substituted by 1 or 2 groups selected from a C₁₋₄ alkyl, =O, a S(O)₂C₁₋₄ alkyl, a C(O)C₃₋₇ cycloalkyl and a C(O)C₁₋₄ alkyl; R₁₀ and R₁₁ each independently represent hydrogen or a C₁₋₄ alkyl group;
X is nitrogen atom, and Y is CH, or X is CH, and Y is nitrogen;
m is 0 or an integer of 1 to 3;
n is an integer of 1 to 3;
p is 0, 1 or 2,
   or a pharmaceutically acceptable salt and a solvate thereof.

Moreover, WO 2003/099787, there is described a piperidine derivative represented by the formula: 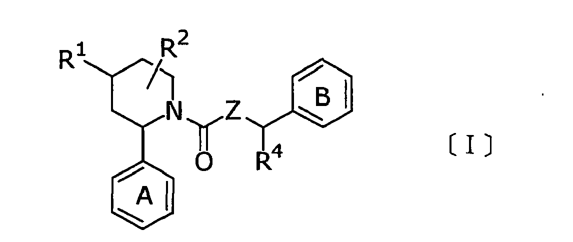 wherein Ring A represents an optionally substituted benzene ring, Ring B represents an optionally substituted benzene ring, R¹ represents an optionally substituted alkyl group, an optionally substituted hydroxyl group, a substituted thiol group, a substituted carbonyl group, a substituted sulfinyl group, a substituted sulfonyl group, or a group represented by the formula: 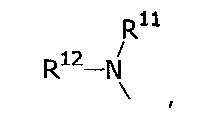 R¹¹ and R¹² may be the same or different from each other, and each represent hydrogen atom, a substituted carbonyl group, a substituted sulfonyl group, an optionally substituted alkyl group or a heterocyclic group having 1 to 4 atoms selected from nitrogen atom, oxygen atom and sulfur atom as a hetero atom(s), the said heterocyclic group may be optionally substituted, and further the nitrogen atom contained in the said heterocyclic group may be oxidized, or may be bonded at their ends to each other with the adjacent nitrogen atom to form a heterocyclic group selected from piperidino group, azacycloheptyl group, pyrrolidino group, imidazolidinyl group, hexahydropyrimidinyl group, thiazolidyl group, morpholino group, triazolyl group, tetrazolyl group and purinyl group, the said heterocyclic group may be optionally substituted, and further the nitrogen atom contained in the said heterocyclic group may be oxidized, R² represents hydrogen atom, an optionally substituted hydroxyl group, an optionally substituted amino group, an optionally substituted alkyl group, a substituted carbonyl group or a halogen atom, Z represents oxygen atom or a group represented by -N(R³)-, R³ represents hydrogen atom or an optionally substituted alkyl group, R⁴ represents hydrogen atom or an optionally substituted alkyl group,
or a pharmaceutically acceptable salt thereof.

### [Disclosure of the invention]

### [Problems to be solved by the invention]

Currently, as a therapeutic agent for the above-mentioned various diseases (especially for emesis, depression, urinary disorder, etc.), there have not been discovered yet any compound having an excellent tachykinin receptor antagonistic action (specifically, SP receptor antagonistic action), and having sufficiently satisfying safety and sustainability (metabolism, dynamics in vivo, and absorption), etc. Therefore, a compound has been sought for which has an excellent tachykinin receptor antagonistic action, and has sufficiently satisfying clinical effect as the therapeutic agent.

### [Means for solving the problems]

The present invention relates to a thiomorpholine compound represented by the formula [I]: 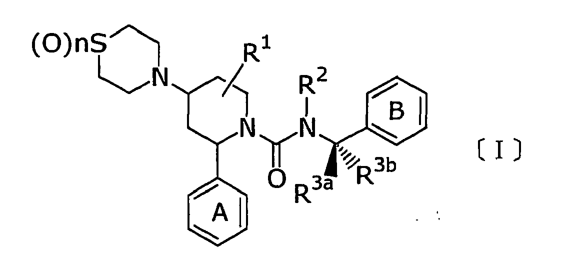 wherein Ring A represents an optionally substituted benzene ring;
Ring B represents an optionally substituted benzene ring;
R¹ represents hydrogen atom, an optionally substituted hydroxyl group, an optionally substituted amino group, an optionally substituted alkyl group, a substituted carbonyl group or a halogen atom; R² represents hydrogen atom or an optionally substituted alkyl group;
R^{3a} and R^{3b} may be the same or different from each other, and each represent hydrogen atom or an optionally substituted alkyl group, or combined to each other at the both ends thereof to form an alkylene group, and
n is an integer of 1 or 2,
or a pharmaceutically acceptable salt thereof.

### [Effects of the invention]

The present invention is to provide a compound having an excellent tachykinin receptor antagonistic action, and has sufficiently satisfying clinical effects such as safety and sustainability (metabolism, dynamics in vivo, and absorption), etc.

### [Best mode to carry out the invention]

In the present invention, Ring A represents an optionally substituted benzene ring, and the substituent(s) of the benzene ring may be mentioned an alkyl group, a halogen atom, cyano group, an optionally protected hydroxyl group or an alkoxy group. Ring A may have the same or different 1 to 3 substituents selected from these substituents.

In the present invention, Ring B represents an optionally substituted benzene ring, and the substituent(s) of the benzene ring may be mentioned a trihalogenoalkyl group, a halogen atom, cyano group, phenyl group, a heterocyclic group having 1 to 4 hetero atoms selected from nitrogen atom, oxygen atom and sulfur atom, an alkyl group, an optionally protected hydroxyl group or an alkoxy group. Ring B may have the same or different 1 to 3 substituents selected from these substituents.

Preferred examples of Ring A and Ring B in the compound of the present invention, there may be mentioned a compound in which, for example, Ring A is a benzene ring represented by the formula: 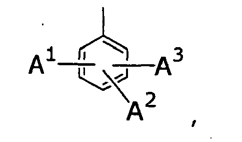 Ring B is a benzene ring represented by the formula: 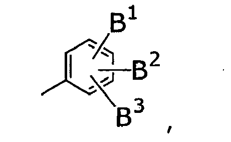 A¹, A² and A³ may be the same or different from each other, and each represent hydrogen atom, a halogen atom, an alkyl group, an optionally protected hydroxyl group or an alkoxy group, B¹, B² and B³ may be the same or different from each other, and each represent hydrogen atom, a trihalogenoalkyl group, a halogen atom, cyano group, phenyl group, a heterocyclic group having 1 to 4 atoms selected from nitrogen atom, oxygen atom and sulfur atom as a hetero atom(s), an alkyl group, an optionally protected hydroxyl group or an alkoxy group. As the trihalogenoalkyl group, there may be mentioned, for example, trifluoromethyl group or trichloromethyl group, etc. As the heterocyclic group having 1 to 4 atoms selected from nitrogen atom, oxygen atom and sulfur atom as a hetero atom(s), there may be mentioned, for example, tetrazolyl group.

In the present invention, as the protective group for the optionally protected hydroxyl group, there may be mentioned a conventionally used protective group such as an optionally substituted arylalkyl group, an optionally substituted silyl group, an acyl group, etc. Of these, preferred may be mentioned, for example, an arylalkyl group such as benzyl group, phenethyl group, etc.; a substituted silyl group such as tert-butyldimethylsilyl group, tert-butyldiphenylsilyl group, etc.; an acyl group such as formyl group, acetyl group, propionyl group, malonyl group, acryloyl group, benzoyl group, etc.

In the present invention, as R¹, there may be mentioned hydrogen atom, an optionally substituted hydroxyl group, an optionally substituted amino group, an optionally substituted alkyl group, a substituted carbonyl group or a halogen atom.
In the present invention, as a substituent of the optionally substituted hydroxyl group of R¹, there may be mentioned an alkyl group.
In the present invention, as a substituent of the optionally substituted amino group of R¹, there may be mentioned an alkyl group.
In the present invention, as a substituent of the optionally substituted alkyl group of R¹, there may be mentioned an alkoxy group.
In the present invention, as a substituent of the substituted carbonyl group of R¹, there may be mentioned hydroxyl group, an alkoxy group or an alkylamino group.
In the present invention, as R², there may be mentioned hydrogen atom or an optionally substituted alkyl group. As a substituent of the optionally substituted alkyl group of R², there may be mentioned hydroxyl group, an alkanoyl group, a halogen atom, an alkoxy group or alkylamino group.
In the present invention, R^{3a} and R^{3b} may be the same or different from each other, and each represent hydrogen atom or an optionally substituted alkyl group, or a group which forms an alkylene group by bonding at the both ends. As a substituent of the optionally substituted alkyl group, there may be mentioned hydroxyl group, etc.
In the present invention, n may be mentioned an integer of 1 or 2.

As Compound [I] of the present invention, preferred is a compound in which Ring A is a benzene ring represented by the formula: 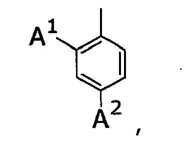 A¹ represents an alkyl group, A² represents a halogen atom, Ring B is a benzene ring represented by the formula: 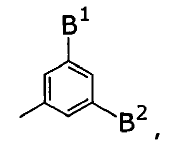 B¹ represents a trihalogenoalkyl group, B² represents a trihalogenoalkyl group, R¹ represents hydrogen atom, R² represents an alkyl group, R^{3a} and R^{3b} may be the same or different from each other, and each represent hydrogen atom or an alkyl group. Also, as Compound [I] of the present invention, preferred is a compound in which n is 2.

Also, in the compounds of the present invention, preferred compounds are a compound selected from the following (a) to (g) or a pharmaceutically acceptable salt thereof.
(a) 1,1-Dioxo-4-(S)-[1-[N-{1-(R)-(3,5-bistrifluoromethylphenyl)ethyl}-N-methyl]-2-(R)-(4-fluoro-2-methylphenyl) - aminocarbonylpiperidin-4-yl]thiomorpholine,
(b) 1,1-Dioxo-4-(R)- [1- [N-{1-(R)-(3,5-bistrifluoromethylphenyl)ethyl}-N-methyl]-2-(R)-(4-fluoro-2-methylphenyl)-aminocarbonylpiperidin-4-yl]thiomorpholine,
(c) 1,1-Dioxo-4-(S)-[1-[N-{1-(S)-(3,5-bistrifluoromethylphenyl)ethyl}-N-methyl]-2-(R)-(4-fluoro-2-methylphenyl)-aminocarbonylpiperidin-4-yl]thiomorpholine,
(d) 1,1-Dioxo-4-(R)-[1-[N-{1-(S)-(3,5-bistrifluoromethylphenyl) ethyl}-N-methyl] -2- (R) - (4-fluoro-2-methylphenyl) - aminocarbonylpiperidin-4-yl]thiomorpholine,
(e) 1,1-Dioxo-4-(S)-[1-{N-(3,5-bistrifluoromethylphenyl)-methyl-N-methyl}-2-(R)-(4-fluoro-2-methylphenyl)aminocarbonylpiperidin-4-yl]thiomorpholine,
(f) 1,1-Dioxo-4-(R)- [1-{N-(3,5-bistrifluoromethylphenyl)-methyl-N-methyl}-2-(R)-(4-fluoro-2-methylphenyl)aminocarbonylpiperidin-4-yl]thiomorpholine and
(g) 4-(S)-[1-[N-{1-(R)-(3,5-bistrifluoromethylphenyl)-ethyl}-N-methyl]-2-(R)-(4-fluoro-2-methylphenyl)amino-carbonylpiperidin-4-yl]-1-oxothiomorpholine.

The Compound [I] of the present invention can be used for a pharmaceutical use either in a free form or in form of a pharmaceutically acceptable salt.
As the pharmaceutically acceptable salt of the Compound [I] of the present invention, there may be mentioned, for example, an inorganic acid salt such as hydrochloride, sulfate, phosphate and hydrobromide; and an organic acid salt such as acetate, fumarate, oxalate, citrate, methanesulfonate, benzenesulfonate, tosylate, maleate, succinate and tartarate.
Further, the Compound [I] of the present invention or a pharmaceutically acceptable salt thereof includes any of its internal salts, solvates and hydrates, etc.
Although an optical isomer based on an asymmetric carbon can be present in the Compound [I] of the present invention, the present invention includes any of these optical isomers and the mixture thereof. In the present invention, among these optical isomers, preferred is a compound having R configuration at 2-position of the piperidine ring (the connecting position of Ring A).

The Compound [I] or a pharmaceutically acceptable salt thereof of the present invention has an excellent tachykinin receptor antagonistic action, particularly an SP receptor antagonistic action, whereby it is useful as a safe medicament for prophylaxis and treatment for inflammation or allergic diseases (for example, atopic dermatitis, dermatitis, herpes, proriasis, asthma, bronchitis, expectoration, rhinitis, rheumatoid arthritis, osteoarthritis, osteoporosis, multiple sclerosis, conjunctivitis, ophthalmia, cystitis, etc.), pain, migraine, neuralgia, itchiness, cough, and further central nervous system diseases (for example, schizophrenia, Parkinson's disease, depression, uneasiness, psychosomatic disorder, morphine dependence, dementia (for example, Alzheimer's disease, etc.), etc.), digestive organs disease (for example, irritable bowel syndrome, ulcerative colitis, Crohn's disease, disorder (for example, gastritis, gastric ulcer, etc.) related to urease-positive *Spirillum* (for example, *helicobacter pylori, etc.),* etc.), nausea, emesis, urinary disorder (for example, pollakiurea, urinary incontinence, etc.), circulatory disease (for example, angina pectoris, hypertension, cardiac failure, thrombosis, etc.) and immune disorder, etc. in mammals (for example, mouse, guinea pig, Mongolian gerbil, ferret, rat, hamster, rabbit, cat, dog, bovine, sheep, monkey, human, etc.). Particularly, since Compound [I] or a pharmaceutically acceptable salt thereof which is an active ingredient of the present invention has a high penetration to the brain and has a low toxicity (high safety), showing almost no side effect, it is useful as a therapeutic or prophylactic agent for central nervous system diseases such as emesis, depression and so forth, or urinary disorder such as pollakiuria, etc.

Measurements on the compound of the present invention or a pharmaceutically acceptable salt thereof can be carried out, according to the method described in European Journal of Pharmacology, vol. 254, pages 221-227 (1994) with respect to a neurokinin-1 receptor binding action, according to the method described in European Journal of Pharmacology, vol. 265, pages 179-183 (1994) with respect to neurokinin-1 receptor antagonistic action, and according to the method described in Journal of Urology, vol. 155, No. 1, pages 355-360 (1996) with regard to an inhibitory action on pollakiuria.

The Compound [I] or a pharmaceutically acceptable salt thereof of the present invention can be administered orally or parenterally, and it can be formulated into a suitable preparation by using a conventionally used pharmaceutical carrier for an oral or parenteral administration. As such a pharmaceutical carrier, there may be mentioned, for example, a binder (syrup, Gum Arabic, gelatin, sorbitol, tragacanth, polyvinylpyrrolidone, etc.), an excipient (lactose, sugar, corn starch, potassium phosphate, sorbitol, glycine, etc.), a lubricant (magnesium stearate, talc, polyethylene glycol, silica, etc.), a disintegrator (potato starch, etc.) and a wetting agent (anhydrous lauryl sodium sulfate, etc.), and the like. Also, when these pharmaceutical preparations are administered orally, they may be a solid preparation such as tablets, granules, capsules and powders, or a liquid preparation such as solution, suspension and emulsion. On the other hand, when they are administered parenterally, for example, they can be administered as an injection solution or an infusion solution by using distilled water for injection, physiological saline, aqueous glucose solution, etc., or they may be administered as a suppository, and the like.

A dose of the Compound [I] or a pharmaceutically acceptable salt thereof of the present invention may vary depending on an administration method, an age, a body weight or a condition of a patient, etc., and, for example, in case of oral administration, it is usually administered in a dose of 0.01 to 20 mg/kg per day, and particularly preferably 0.01 to 10 mg/kg per day, and in case of parenteral administration, usually in a dose of 0.01 to 10 mg/kg per day, particularly preferably 0.01 to 1 mg/kg per day.

### [Method A]

The objective Compound [I] of the present invention can be prepared, for example, by reacting a compound represented by the formula [II] : 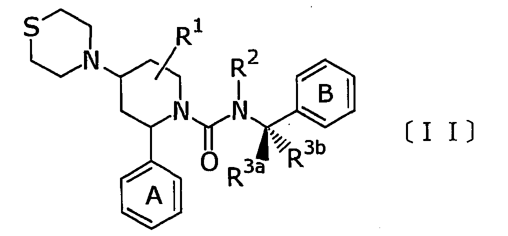 wherein Ring A represents an optionally substituted benzene ring;
Ring B represents an optionally substituted benzene ring;
R¹ represents hydrogen atom, an optionally substituted hydroxyl group, an optionally substituted amino group, an optionally substituted alkyl group, a substituted carbonyl group or a halogen atom;
R² represents hydrogen atom or an optionally substituted alkyl group; and
R^{3a} and R^{3b} may be the same or different from each other, and each represent hydrogen atom or an optionally substituted alkyl group, or a group which forms
an alkylene group at the ends thereof,
in the presence of an oxidizing agent. -

### [Method B]

The objective Compound [I] of the present invention can be prepared, for example, by reacting a compound represented by the formula [III]: 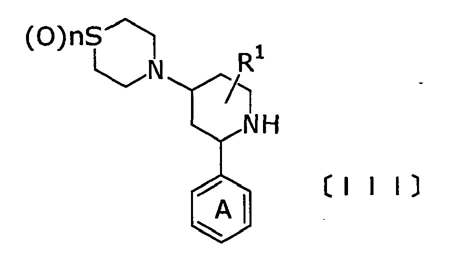 wherein Ring A, R¹ and n have the same meanings as defined above,
and a compound represented by the formula [IV] : 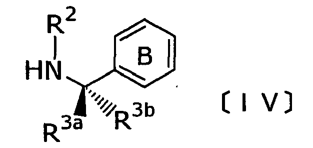 wherein Ring B, R², R^{3a} and R^{3b} have the same meanings as defined above,
in the presence of a urea bond forming agent.

### [Method C]

The objective Compound [I] of the present invention can be prepared, for example, by reacting a compound or a salt thereof represented by the formula [V]: 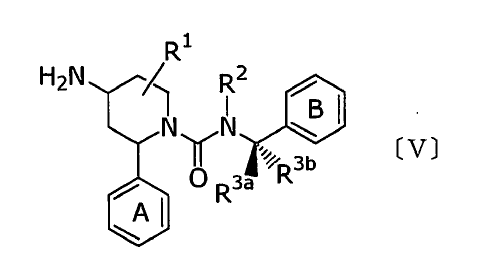 wherein Ring A, Ring B, R¹, R², R^{3a} and R^{3b} have the
same meanings as defined above,
and a compound represented by the formula [VI]: 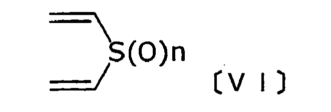 wherein n has the same meanings as defined above, in the presence of a base.

These [Method A] to [Method C] can be carried out as follows.

### [Method A]

This preparation method can be carried out by reacting Compound [II] in the presence of an oxidizing agent, in a suitable solvent. As the oxidizing agent, there may be mentioned, for example, 3-chloroperbenzoic acid, peracetic acid, sodium periodate, OXONE, etc. Also, as the solvent, any solvent may be used so long as it does not exert any bad effect on the reaction, and, for example, dichloromethane, chloroform, acetonitrile, dimethoxyethane, tetrahydrofuran, water, etc. may be optionally used. This reaction can be carried out, for example, at -80°C to 150°C, preferably at 0°C to 50°C.

### [Method B]

The reaction of Compound [III] and Compound [IV] can be carried out in the presence of a urea bond forming agent, in a suitable solvent. As the urea bond forming agent, there may be mentioned a compound represented by the formula: 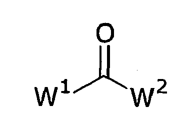 wherein W¹ and W² may be the same or different from
each other, and each represent a leaving group. W¹ and W² may be the same or different and each may be mentioned imidazolyl group, a halogen atom or phenoxy group. Specifically, 1,1'-carbonyldiimidazole, phosgene, etc. are preferred, and for example, carbonyl dihalide such as 1,1'-carbonyldiimidazole, triphosgene or phosgene, etc. can be used. Also, as the solvent, any solvent may be used so long as it does not exert any bad effect on the reaction, and, for example, acetonitrile, dichloromethane, tetrahydrofuran, etc. may be optionally used. This reaction can be carried out, for example, at 0°C to 80°C, preferably at 0°C to 50°C.
Moreover, this reaction can be carried out by reacting Compound [III] and a urea bond forming agent represented by the formula: 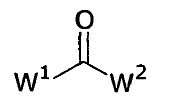 wherein W¹ and W² have the same meanings as defined above,
to prepare a compound represented by the formula [VII]: 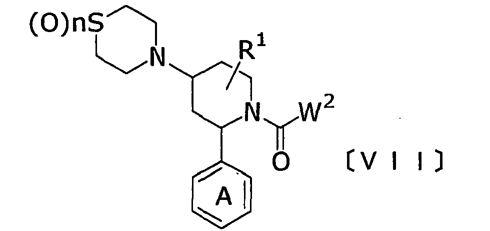 wherein Ring A, R¹, W² and n have the same meanings as defined above,
then, Compound [VII] is led to its reactive derivative, and reacting it with Compound [IV], or reacting Compound [IV] and a urea bond forming agent represented by the formula: 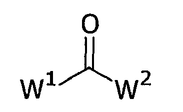 wherein W¹ and W² have the same meanings as defined above,
to prepare a compound represented by the formula [VIII]: 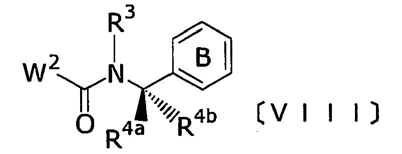 wherein Ring B, R², R^{3a}, R^{3b} and W² have the same meanings as defined above,
then, Compound [VIII] is led to its reactive derivative, and reacting it with Compound [III] to prepare Compound [I].

As the reactive derivatives, there may be mentioned, for example, in Compound [VII] or Compound [VIII], a compound in which W² is led to a group represented by the formula: 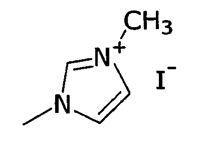 may be mentioned.

The reaction between Compound [III] or Compound [IV] and the urea bond forming agent can be carried out, for example, at 0°C to 80°C, preferably at 0°C to 50°C. Also, as the solvent, any solvent may be used so long as it does not exert any bad effect on the reaction, and, for example, acetonitrile, dichloromethane, tetrahydrofuran, etc. may be optionally used.

A reaction that lead Compound [VII] or Compound [VIII] to its reactive derivatives can be carried out, for example, by using a reactive derivatizing agent such as methyl iodide at 0°C to 80°C, preferably at 0°C to 50°C. Also, as the solvent, any solvent may be used so long as it does not exert any bad effect on the reaction, and, for example, acetonitrile, dichloromethane, tetrahydrofuran, etc. may be optionally used.

The reaction of the respective reactive derivatives and Compound [III] or Compound [IV] can be carried out, for example, in the presence of a base at 0°C to 80°C, preferably at 0°C to 50°C. Also, as the base, there may be used any material, for example, triethylamine, etc., and as the solvent, it may be used any material so long as it does not exert any bad effect on the reaction may be used, and there may be optionally used, for example, toluene, acetonitrile, dichloromethane, tetrahydrofuran, etc.

### [Method C]

The reaction of Compound [V] and Compound [VI] can be carried out in the presence of a base, in a suitable solvent. As the base, there may be mentioned, for example, an organic base such as triethylamine, diisopropylethylamine, tri-n-butylamine, pyridine, 1,8-diazabicyclo-[5.4.0]undec-7-ene, etc. or inorganic base such as potassium carbonate, sodium carbonate, cesium carbonate, potassium hydroxide, sodium hydroxide, sodiume methoxide, potassium tert-butoxide, etc. Also, as the solvent, any solvent may be used so long as it does not exert any bad effect on the reaction, and, for example, methanol, ethanol, isopropyanol, N,N-dimethylformamide, 1,4-dioxane, tetrahydrofran, ethyl acetate, toluene, etc. or the mixed solvent thereof, or the mixture of the solvent or the mixed solvent thereof and water may be optionally used. As the salt of the compound [V], there may be mentioned, for example, an inorganic acid salt such as hydrochloride, hydrobromide, sulfate, etc. and an organic acid salt such as methanesulfonate, p-toluen sulfonate, acetate, carbonate, trifluoroacetate, etc. This reaction can be carried out, for example, at 10°C to under reflux, preferably at 50°C to 120°C.
Incidentally, the compound represented by the formula [IX]: 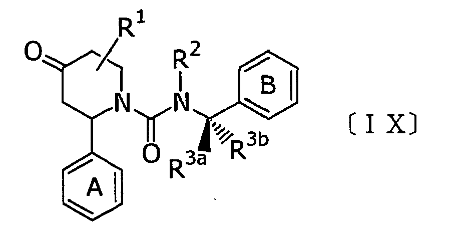 wherein Ring A, Ring B, R¹, R², R^{3a} and R^{3b} have the same meanings as defined above,
or its optical isomer can be prepared according to the method as disclosed in WO 2003/099787.

Compound [II] can be obtained by subjecting Compound [IX] or a salt thereof and thiomorpholine in a suitable solvent to reductive amination. This reductive amination can be carried out under acidic conditions by effecting hydrogenation with a reducing agent such as sodium borohydride, sodium triacetoxy borohydride, sodium cyanoborohydride, etc. or a reducing catalyst such as palladium, etc. As the solvent, any solvent may be used so long as it does not exert any bad effect on the reaction, and, for example, dichloromethane, acetic acid, ethanol, methanol, etc. may be optionally used. As a salt of Compound [IX], hydrochloride, acetate, etc. may be optionally used. This reaction can be carried out, for example, at -10°C to 80°C, preferably at 0°C to 30°C.

Also, the compound represented by the formula [X]: 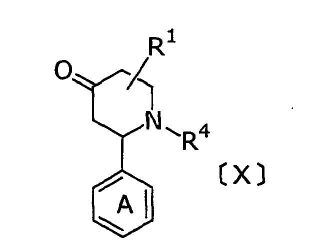 wherein R⁴ represents hydrogen atom or a protective group for amino group, Ring A and R¹ have the same meanings as defined above,
or its optical isomer can be prepared according to the method as disclosed in WO 2002/032867.
As the protective group for the amino group of R⁴, there may be mentioned an alkoxycarbonyl group such as tert-butoxycarbonyl group, etc., and an arylalkoxycarbonyl group such as benzyloxycarbonyl group, etc.

Compound [III] can be obtained by subjecting Compound [X] or a salt thereof and thiomorpholine to reductive amination in a suitable solvent, and when R⁴ is a protective group for amino group, the protective group is then removed. The reductive amination can be carried out under acidic conditions by effecting hydrogenation with a reducing agent such as sodium borohydride, triacetoxy sodium borohydride, sodium cyanoborohydride, etc. or a reducing catalyst such as palladium, etc. As the solvent, any solvent may be used so long as it does not exert any bad effect on the reaction, and, for example, dichloromethane, acetic acid, ethanol, methanol, etc. may be optionally used. As a salt of Compound [X], hydrochloride, acetate, etc. may be optionally used. This reaction can be carried out, for example, at -10°C to 80°C, preferably at 0°C to 30°C. Also, removal of the protective group of the amino group can be carried out according to the conventional manner.

Furthermore, the compound represented by the formula [XI] : 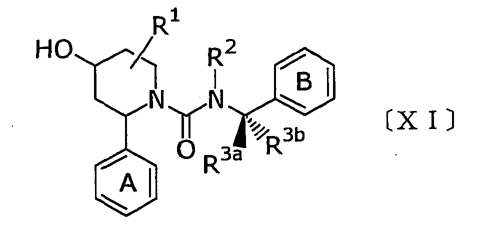 wherein Ring A, Ring B, R¹, R², R^{3a} and R^{3b} have the same meanings as defined above,
or its optical isomer thereof can be prepared by the method disclosed in WO 2003/099787.

The compound represented by the formula [XII]: 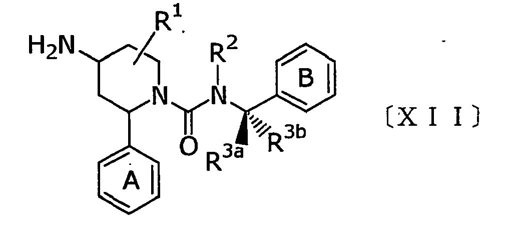 wherein Ring A, Ring B, R¹, R², R^{3a} and R^{3b} have the same meanings as defined above,
or its optical isomer thereof can be prepared by the method disclosed in WO 2003/099787.
Also, the Compound [III] can be prepared by the same 15 manner of the above Method C, for example, by reacting a compound or a salt thereof represented by the formula [XIII] : 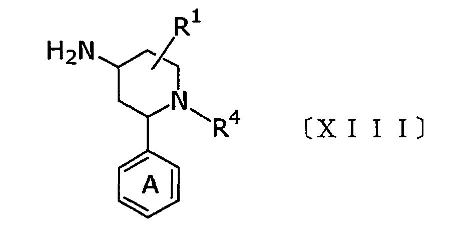 wherein Ring A, R¹ and R⁴ have the same meanings as defined above,
and a compound represented by the formula [VI]: 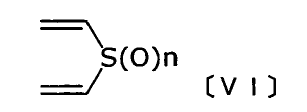 wherein n has the same meanings as defined above, in the presence of a base, and then, removing the protective group of an amino group (R⁴), if desired.

Moreover, for preparing the objective compounds and starting compounds of the present invention, when the starting compounds or the respective intermediate compounds have a functional group(s), a suitable protective group is introduced into the respective functional group(s) according to the conventional manner of synthetic chemistry other than those as mentioned above, and, if they are not necessary, these protective groups may be optionally removed.

In the present specification, the alkyl group means, for example, a straight or branched alkyl group having 1 to 6 carbon atoms such as methyl group, ethyl group, propyl group, isopropyl group, butyl group, isobutyl group, tert-butyl group, isopentyl group, etc., preferably those having 1 to 4 carbon atoms. The alkenyl group means, for example, a straight or branched alkenyl group having 2 to 7 carbon atoms such as vinyl group, allyl group, propenyl group, isopropenyl group, etc., preferably those having 2 to 5 carbon atoms. The alkoxy group means, for example, a straight or branched alkoxy group having 1 to 6 carbon atoms such as methoxy group, ethoxy group, propoxy group, isopropoxy group, butoxy group, etc., preferably those having 1 to 4 carbon atoms. The alkanoyl group means, for example, a straight or branched alkanoyl group having 1 to 6 carbon atoms such as formyl group, acetyl group, propionyl group, butyryl group, valeryl group, tert-butylcarbonyl group, etc., preferably those having 1 to 4 carbon atoms. The cycloalkyl group means, for example, a cycloalkyl group having 3 to 8 carbon atoms such as cyclopropyl group, cyclobutyl group, cyclopentyl group, cyclohexyl group, cycloheptyl group, cyclooctyl group, etc., preferably those having 3 to 6 carbon atoms. Further, the halogen atom is exemplified by chlorine atom, bromine atom, fluorine atom or iodine atom.

### EXAMPLE 5

### Example 1

To 2 ml of a dichloromethane solution containing 90 mg of 4- (S) - [1- [N-{1- (R) - (3, 5-bistrifluoromethylphenyl) - ethyl}-N-methyl]-2-(R)-(4-fluoro-2-methylphenyl)aminocarbonylpiperidin-4-yl]thiomorpholine were added 0.02 ml of methanesulfonic acid and 105 mg of meta-chloroperbenzoic acid, and the mixture was stirred at room temperature for 3 hours. To the reaction mixture was added a sodium thiosulfate aqueous solution, and the resulting mixture was stirred at room temperature for 30 minutes. To the mixture were added water and chloroform, the liquids were separated, and the aqueous layer was again extracted with chloroform. The combined organic layers were dried over anhydrous magnesium sulfate, and concentrated under reduced pressure. The obtained residue was purified by basic silica gel column chromatography (n-hexane:ethyl acetate= 3:1), and then, by silica gel column chromatography (chloroform:ethyl acetate=3:1) to obtain 44 mg of 1,1-dioxo-4-(S)-[1-[N-{1-(R)-(3,5-bistrifluoromethylphenyl)-ethyl}-N-methyl]-2-(R)-(4-fluoro-2-methylphenyl)amino-carbonylpiperidin-4-yl]thiomorpholine shown in the following Table 1.

### Examples 2 to 6

By using the corresponding starting compounds, the same procedures as in Example 1 were carried out to obtain the compounds shown in the following Tables 1 and 2.

### Example 7

To 5 ml of a dichloromethane solution containing 80 mg of 4-(S)-[1-[N-{1-(R)-(3,5-bistrifluoromethylphenyl)-ethyl}-N-methyl]-2-(R)-(4-fluoro-2-methylphenyl)amino-carbonylpiperidin-4-yl]thiomorpholine was added dropwise 0.034 ml of 4M hydrochloric acid (1,4-dioxane solution), then, 33 mg of meta-chloroperbenzoic acid (70%) was added to the mixture at -10°C and the resulting mixture was stirred for one hour. To the reaction mixture were added an aqueous sodium hydrogen carbonate solution and dichloromethane, and then, the liquids were separated. The obtained organic layer was dried over anhydrous sodium sulfate, the solvent was removed under reduced pressure and the residue was purified by silica gel column chromatography (chloroform: methanol=19:1). The product was lyophilized with tert-butanol to obtain 50 mg of 4-(S)-[1-[N-{1-(R)-(3,5-bistrifluoromethylphenyl)ethyl}-N-methyl]-2-(R)-(4-fluoro-2-methylphenyl)aminocarbonylpiperidin-4-yl]-1-oxothiomorpholine shown in the following Table 2.

### Example 8

To 15 ml of a 1,4-dioxane solution containing 11.3 g of (2R,4S)-1-[N-{1-(R)-(3,5-bistrifluoromethylphenyl)-ethyl}-N-methyl]aminocarbonyl-4-tert-butoxycarbonylamino-2-(4-fluoro-2-methylphenyl)piperidine was added dropwise 45 ml of 4M hydrochloric acid (1,4-dioxane solution), and the resulting mixture was stirred for two hours at room temperature. The solvent was removed under reduced pressure and the residue was dried under vacuum. To 50 ml an ethanol solution containing the residue was added 3 g of divinyl sulfone and 5.1 g of triethylamine, and the resulting mixture was stirred for two and half hours under reflux. The solvent was removed under reduced pressure and the residue was crystallized by diisopropylether to obtain 10.3 g of the same compound shown in the above-mentioned Example 1.

### Reference Example 1

To 60 ml of a tetrahydrofuran solution containing 3.91 g of N-{1-(S)-(3,5-bistrifluoromethylphenyl)ethyl}-N-methylamine was added 2.34 g of 1,1'-carbonyldiimidazole, and the mixture was stirred at 40°C overnight. After the solvent was distilled off from the reaction mixture, ethyl acetate was added to the residue, and the whole organic layers were washed with water and saturated saline solution, dried and the solvent was distilled off. The residue was crystalized with diisopropyl ether and collected by filtration. The obtained white crystals were dissolved in 60 ml of acetonitrile, then, 3.4 ml of methyl iodide was added to the solution and the mixture was reacted at 60°C for 2 hours, and the solvent was distilled off. The residue was dissolved in 40 ml of dichloromethane, and under ice-cooling, 3.47 g of 2-(4-fluoro-2-methylphenyl)-4,4-dimethoxypiperidine and 3.82 ml of triethylamine were added to the solution and the mixture was stirred at room temperature overnight. The reaction mixture was poured into water, the liquids were separated, and the aqueous layer was extracted with dichloromethane. The whole organic layers were washed with water and a saturated saline solution, dried and the solvent was distilled off. The residue was dissolved in 90 ml of tetrahydrofuran, and under ice-cooling, 30 ml of 1M aqueous sulfuric acid solution was added to the solution, and the mixture was stirred at room temperature for 5 hours. After adjusting the pH of the mixture to 8 to 9 by using 1M aqueous sodium hydroxide solution, tetrahydrofuran was distilled off, water and ethyl acetate was added to the residue and the liquids were separated, and the aqueous layer was again extracted with ethyl acetate. The whole organic layers were washed with water and a saturated brine, dried and the solvent was distilled off. The residue was purified by silica gel column chromatography (hexane:ethyl acetate=2:1) to obtain 2.12 g of (2R)-1-[N-{1-(S)-(3,5-bistrifluoromethylphenyl)ethyl}-N-methyl]aminocarbonyl-2-(4-fluoro-2-methylphenyl)-4-oxopiperidine shown in the following Table 3.

### Reference Examples 2 and 3

By using the corresponding starting compounds, the same procedures as in Reference Example 1 were carried out to obtain the compounds shown in the following Table 3.

### Reference Example 4

To 15 ml of a dichloromethane solution containing 504 mg of (2R)-1-[N-{1-(S)-(3,5-bistrifluoromethylphenyl) - ethyl}-N-methyl]aminocarbonyl-2-(4-fluoro-2-methylphenyl)-4-oxopiperidine, 155 mg of thiomorpholine and 0.057 ml of acetic acid was added 446 mg of triacetoxy sodium borohydride and the mixture was stirred at room temperature for 16 hours. To the reaction mixture were added 52 mg of thiomorpholine and 112 mg of triacetoxy sodium borohydride and the mixture was further stirred at room temperature for 3 hours. Water and chloroform were added to the mixture, the liquids were separated, and the aqueous layer was again extracted with chloroform. The combined organic layers were dried over anhydrous magnesium sulfate, and concentrated under reduced pressure. The obtained residue was purified by basic silica gel column chromatography (n-hexane:ethyl acetate=3:1), and then, by silica gel column chromatography (chloroform:ethyl acetate=3:1) to obtain 92 mg of (a) 4-(S)-[2-(R)-(4-fluoro-2-methylphenyl)-1-[N-{1-(R)-(3,5-bistrifluoromethylphenyl)ethyl}-N-methyl]aminocarbonylpiperidin-4-yl]thiomorpholine and 164 mg of (b) 4-(R)-[2-(R)-(4-fluoro-2-methylphenyl)-1-[N-{1-(R)-(3,5-bistrifluoromethylphenyl)ethyl}-N-methyl]aminocarbonylpiperidin-4-yl]thiomorpholine shown in the following Table 3.

### Reference Examples 5 and 6

By using the corresponding starting compounds, the same procedures as in Reference Example 4 were carried out to obtain the compounds shown in the following Table 4.

### Reference Example 7

To an N,N-dimethylformamide solution containing 280 mg of (2R,4R)-1-[N-{1-(R)-(3,5-bistrifluoromethylphenyl) - ethyl}-N-methyl]aminocarbonyl-4-bromo-2-(4-fluoro-2-methylphenyl)piperidine was added 0.2 ml of thiomorpholine, and the mixture was stirred at 90°C for 16 hours. The reaction mixture was concentrated under reduced pressure, and the residue was purified by silica gel column chromatography (n-hexane:ethyl acetate=1:3) to obtain 80 mg of the same compound as in the above-mentioned Reference Example 4(a).

[Table 1]

**Table 1**

| Example No. | Structural formula | MS |
|---|---|---|
| 1 | 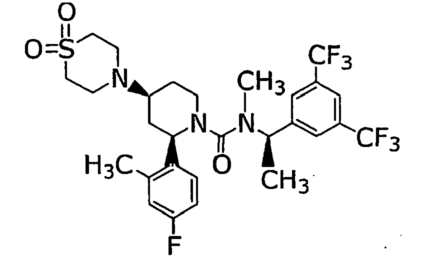 | 624 (M⁺+1) |
| 2 | 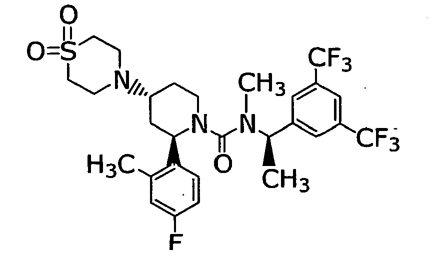 | 624 (M⁺+1) |
| 3 | 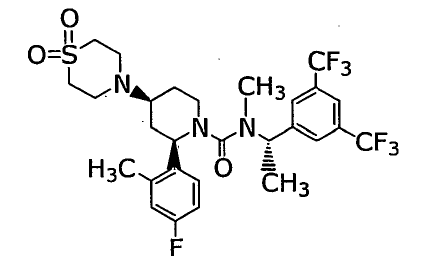 | 624 (M⁺+1) |
| 4 | 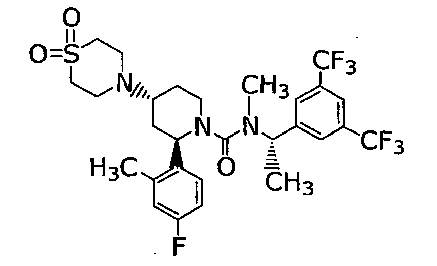 | 624 (M⁺+1) |

[Table 2]

**Table 2**

| Example No. | Structural formula | MS |
|---|---|---|
| 5 | 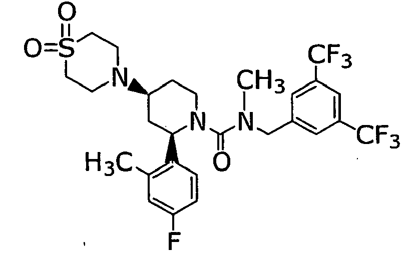 | 610 (M⁺+1) |
| 6 | 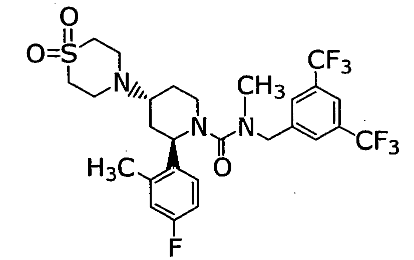 | 610 (M⁺+1) |
| 7 | 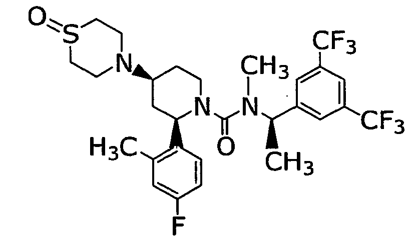 | 608 (M⁺+1) |

[Table 3]

**Table 3**

| Reference Example No. | Structural formula | MS |
|---|---|---|
| 1 | 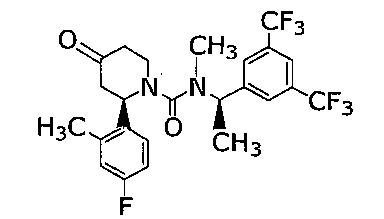 | 505 (M⁺+1) |
| 2 | 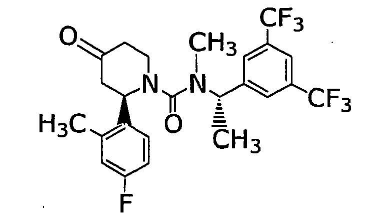 | 505 (M⁺+1) |
| 3 | 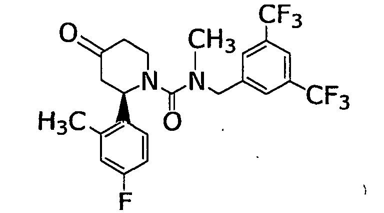 | 491 (M⁺+1) |
| 4 (a) | 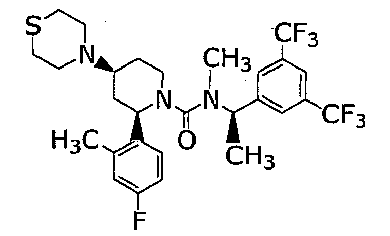 | 592 (M⁺+1) |
| 4 (b) | 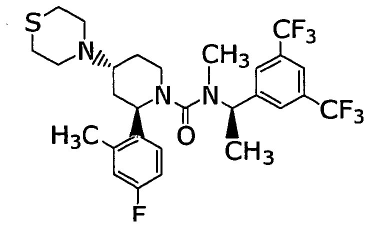 | 592 (M⁺+1) |

[Table 4]

**Table 4**

| Reference Example No. | Structural formula | MS |
|---|---|---|
| 5 (a) | 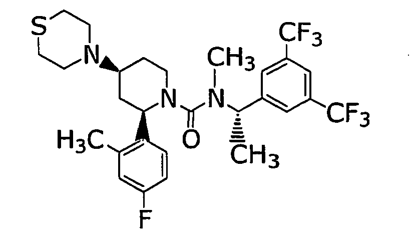 | 592 (M⁺+1) |
| 5 (b) | 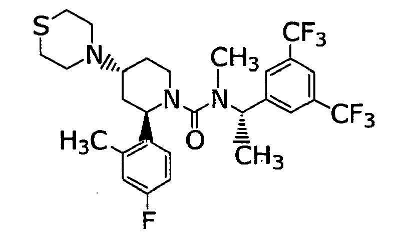 | 592 (M⁺+1) |
| 6 (a) | 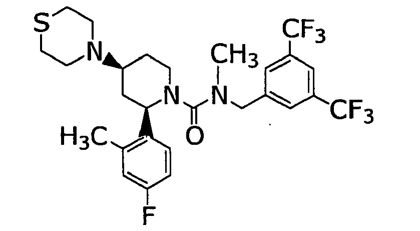 | 578 (M⁺+1) |
| 6 (b) | 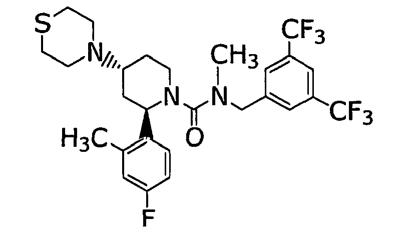 | 578 (M⁺+1) |

### [Utilizability in industry]

The compounds of the present invention have an excellent tachykinin receptor antagonistic action. Also, the compounds of the present invention have high safety, and excellent in the points of absorption, penetration to the brain, stability in metabolism, concentration in blood, sustainability, etc., and thus, have excellent medical effects.

## Claims

1. A thiomorpholine compound represented by the formula [I]: 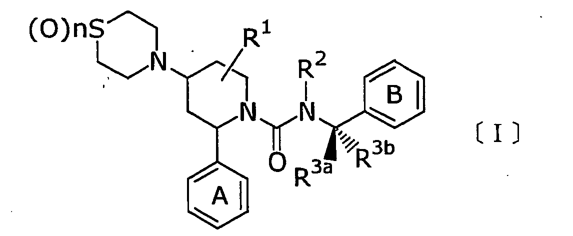 wherein Ring A represents an optionally substituted benzene ring;
Ring B represents an optionally substituted benzene ring;
R¹ represents hydrogen atom, an optionally substituted hydroxyl group, an optionally substituted amino group, an optionally substituted alkyl group, a substituted carbonyl group or a halogen atom;
R² represents hydrogen atom or an optionally substituted alkyl group;
R^{3a} and R^{3b} may be the same or different from each other, and each represent hydrogen atom or an optionally substituted alkyl group, or combined to each other at the both ends thereof to forum an
alkylene group, and
n is an integer of 1 or 2,
or a pharmaceutically acceptable salt thereof.

2. The thiomorpholine compound or a pharmaceutically acceptable salt thereof according to Claim 1,
wherein Ring A is a benzene ring represented by the formula: 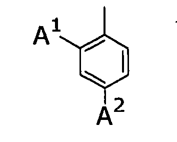 where A¹ is an alkyl group, and A² is a halogen atom, Ring B is a benzene ring represented by the formula: 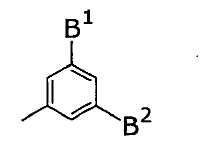 where B¹ represents a trihalogenoalkyl group, and B² represents a trihalogenoalkyl group,
R¹ represents hydrogen atom, R² represents an alkyl group,
R^{3a} and R^{3b} are the same or different from each other, and each represent hydrogen atom or alkyl group.

3. A compound according to claim 1, selected from the following (a) to (g):
(a) 1,1-dioxo-4-(S)-[1-[N-{1-(R)-(3,5-bistrifluoromethylphenyl) ethyl}-N-methyl] -2- (R) - (4-fluoro-2-methylphenyl) - aminocarbonylpiperidin-4-yl]thiomorpholine,
(b) 1,1-dioxo-4-(R)-[1-[N-{1-(R)-(3,5-bistrifluoromethylphenyl)ethyl}-N-methyl]-2-(R)-(4-fluoro-2-methylphenyl) - aminocarbonylpiperidin-4-yl]thiomorpholine,
(c) 1,1-dioxo-4-(S)-[1-[N-{1-(S)-(3,5-bistrifluoromethylphenyl)ethyl}-N-methyl]-2-(R)-(4-fluoro-2-methylphenyl)-aminocarbonylpiperidin-4-yl]thiomorpholine,
(d) 1,1-dioxo-4-(R)-[1-[N-{1-(S)-(3,5-bistrifluoromethylphenyl) ethyl}-N-methyl] -2- (R) - (4-fluoro-2-methylphenyl) - aminocarbonylpiperidin-4-yl]thiomorpholine,
(e) 1,1-dioxo-4-(S)-[1-{N-(3,5-bistrifluoromethylphenyl)-methyl-N-methyl}-2-(R)-(4-fluoro-2-methylphenyl)amino-carbonylpiperidin-4-yl]thiomorpholine,
(f) 1,1-dioxo-4-(R)-[1-{N-(3,5-bistrifluoromethylphenyl)-methyl-N-methyl}-2-(R)-(4-fluoro-2-methylphenyl)amino-carbonylpiperidin-4-yl]thiomorpholine, and
(g) 4-(S)-[1-[N-{1-(R)-(3,5-bistrifluoromethylphenyl)-ethyl}-N-methyl]-2-(R)-(4-fluoro-2-methylphenyl)amino-carbonylpiperidin-4-yl]-1-oxothiomorpholine,
or a pharmaceutically acceptable salt thereof.

4. A process for preparing a thiomorpholine compound represented by the formula [I]: 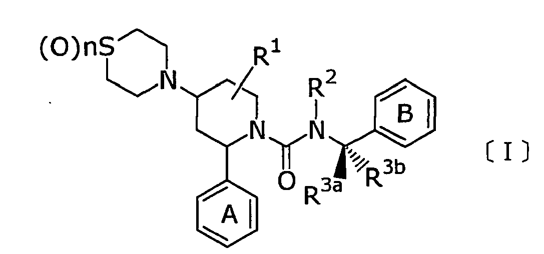 wherein Ring A represents an optionally substituted benzene ring;
Ring B represents an optionally substituted benzene ring;
R¹ represents hydrogen atom, an optionally substituted hydroxyl group, an optionally substituted amino group, an optionally substituted alkyl group, a substituted carbonyl group or a halogen atom;
R² represents hydrogen atom or an optionally substituted alkyl group;
R^{3a} and R^{3b} may be the same or different from each other, and each represent hydrogen atom or an optionally substituted alkyl group, or combined to each other at the both ends thereof to form an alkylene group, and
n is an integer of 1 or 2,
or a pharmaceutically acceptable salt thereof,
which comprises reacting a compound represented by the formula [II] : 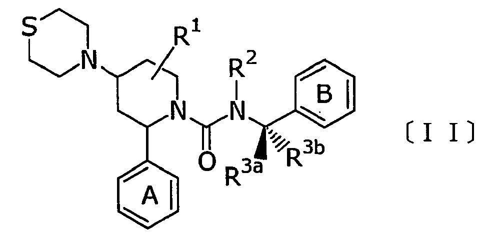 wherein Ring A, Ring B, R¹, R², R^{3a} and R^{3b} have the same meanings as defined above,
in the presence of an oxidizing agent, and then, converting it into a pharmaceutically acceptable salt thereof, if desired.

5. A process for preparing a thiomorpholine compound represented by the formula [I]: 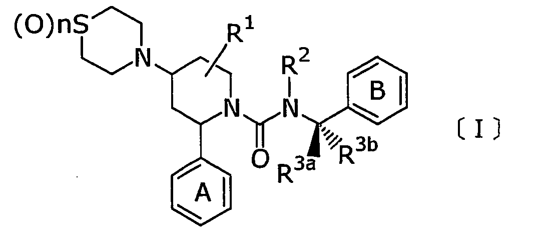 wherein Ring A represents an optionally substituted benzene ring;
Ring B represents an optionally substituted benzene ring;
R¹ represents hydrogen atom, an optionally substituted hydroxyl group, an optionally substituted amino group, an optionally substituted alkyl group, a substituted carbonyl group or a halogen atom;
R² represents hydrogen atom or an optionally substituted alkyl group;
R^{3a} and R^{3b} may be the same or different from each other, and each represent hydrogen atom or an optionally substituted alkyl group, or combined to each other at the both ends thereof to form an alkylene group, and
n is an integer of 1 or 2,
or a pharmaceutically acceptable salt thereof,
which comprises reacting a compound represented by the formula [III]: 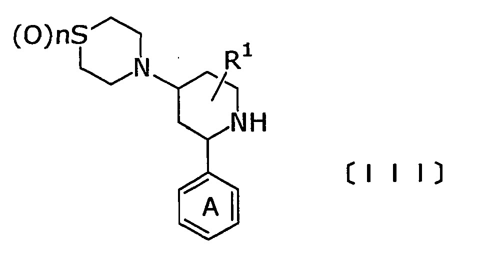 wherein Ring A, R¹ and n have the same meanings as defined above,
and a compound represented by the formula [IV]: 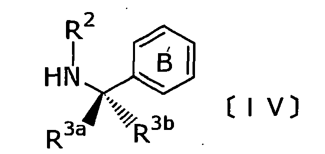 wherein Ring B, R², R^{3a} and R^{3b} have the same meanings as defined above,
in the presence of a urea bond forming agent, and then,
converting it into a pharmaceutically acceptable salt thereof, if desired.

6. A process for preparing a thiomorpholine compound represented by the formula [I]: 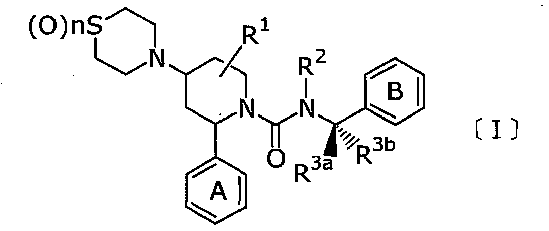 wherein Ring A represents an optionally substituted benzene ring;
Ring B represents an optionally substituted benzene ring;
R¹ represents hydrogen atom, an optionally substituted hydroxyl group, an optionally substituted amino
group, an optionally substituted alkyl group, a substituted carbonyl group or a halogen atom;
R² represents hydrogen atom or an optionally substituted alkyl group;
R^{3a} and R^{3b} may be the same or different from each other, and each represent hydrogen atom or an optionally substituted alkyl group, or combined to each other at the both ends thereof to form an alkylene group, and
n is an integer of 1 or 2,
or a pharmaceutically acceptable salt thereof,
which comprises reacting a compound or a salt thereof
represented by the formula [V]: 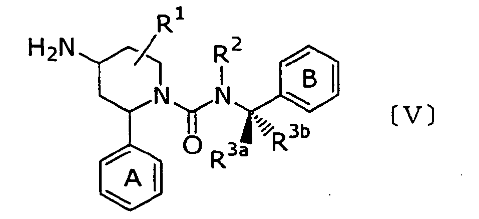 wherein Ring A, Ring B, R¹, R², R^{3a} and R^{3b} have the same meanings as defined above,
and a compound represented by the formula [IV] : 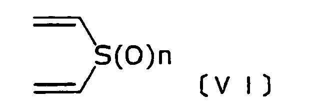 wherein n has the same meanings as defined above,
in the presence of a base, and then, converting it into a pharmaceutically acceptable salt thereof, if desired.

7. A pharmaceutical composition comprising the compound according to any one of Claims 1 to 3, in a clinically effective dose and a pharmaceutically acceptable carrier.

8. The compound according to any one of Claims 1 to 3 for a use as a clinically effective ingredient.

9. Use of the compound according to any one of Claims 1 to 3, for preparation of a medicament for treatment and prophylaxis of a disease selected from inflammation, allergic diseases, pain, migraine, neuralgia, itchiness, cough, central nervous system diseases, digestive organs disease nausea, emesis, urinary disorder, circulatory disease and immune disorder.

10. The use according to Claim 9, wherein the disease is urinary disorder.

## Patentansprüche

1. Thiomorpholinverbindung, dargestellt durch die Formel [I] : 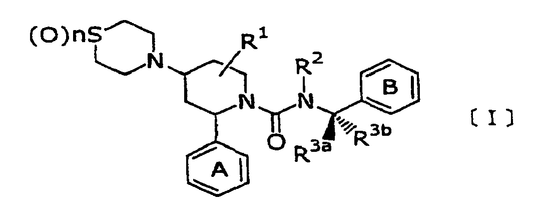 worin der Ring A einen optional substituierten Benzolring darstellt;
der Ring B einen optional substituierten Benzolring darstellt;
R¹ ein Wasserstoffatom, eine optional substituierte Hydroxylgruppe, eine optional substituierte Aminogruppe, eine optional substituierte Alkylgruppe, eine substituierte Carbonylgruppe oder ein Halogenatom darstellt;
R² ein Wasserstoffatom oder eine optional substituierte Alkylgruppe darstellt;
R^{3a} und R^{3b} gleich oder voneinander verschieden sein können und jeweils ein Wasserstoffatom oder eine optional substituierte Alkylgruppe darstellen, oder sie an beiden Enden von ihnen miteinander verbunden sind, um eine Alkylengruppe zu bilden, und
n eine ganze Zahl von 1 oder 2 ist,
oder ein pharmazeutisch akzeptables Salz hiervon.

2. Thiomorpholinverbindung oder ein pharmazeutisch akzeptables Salz hiervon gemäß Anspruch 1, worin der Ring A ein Benzolring ist, der durch die Formel: 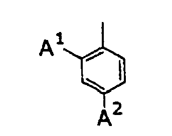 dargestellt wird,
worin A¹ eine Alkylgruppe ist und A² ein Halogenatom ist, worin der Ring B ein Benzolring ist, der durch die Formel 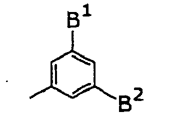 dargestellt wird,
worin B¹ eine Trihalogenalkylgruppe darstellt und B² eine Trihalogenalkylgruppe darstellt,
R¹ ein Wasserstoffatom darstellt, R² eine Alkylgruppe darstellt, R^{3a} und R^{3b} gleich oder voneinander verschieden sind und jeweils ein Wasserstoffatom oder eine Alkylgruppe darstellen.

3. Verbindung gemäß Anspruch 1, ausgewählt aus den folgenden (a) bis (g):
(a) 1,1-Dioxo-4-(S)-[1-[N-{1-(R)-(3,5-bistrifluormethylphenyl)ethyl}-N-methyl]-2-(R)-(4-fluor-2-methylphenyl)aminocarbonylpiperidin-4-yl]thiomorpholin,
(b) 1,1-Dioxo-4-(R)-[1-[N-{1-(R)-(3,5-bistrifluormethylphenyl)ethyl}-N-methyl]-2-(R)-(4-fluor-2-methylphenyl)aminocarbonylpiperidin-4-yl]thiomorpholin,
(c) 1,1-Dioxo-4-(S)-[1-[N-{1-(S)-(3,5-bistrifluormethylphenyl)ethyl}-N-methyl]-2-(R)-(4-fluor-2-methylphenyl)aminocarbonylpiperidin-4-yl]thiomorpholin,
(d) 1,1-Dioxo-4-(R)-[1-[N-{1-(S)-(3,5-bistrifluormethylphenyl)ethyl}-N-methyl]-2-(R)-(4-fluor-2-methylphenyl)aminocarbonylpiperidin-4-yl]thiomorpholin,
(e) 1,1-Dioxo-4-(S)-[1-{N-(3,5-bistrifluormethylphenyl)methyl-N-methyl}-2-(R)-(4-fluor-2-methylphenyl)aminocarbonylpiperidin-4-yl]thiomorpholin,
(f) 1,1-Dioxo-4-(R)-[1-{N-(3,5-bistrifluormethylphenyl)methyl-N-methyl}-2-(R)-(4-fluor-2-methylphenyl)aminocarbonylpiperidin-4-yl]thiomorpholin,
(g) 4-(S)-[1-[N-{1-(R)-(3,5-bistrifluormethylphenyl)ethyl}-N-methyl]-2-(R)-(4-fluor-2-methylphenyl)aminocarbonylpiperidin-4-yl]-1-oxothiomorpholin,
oder pharmazeutisch akzeptablen Salzen hiervon.

4. Verfahren zur Herstellung einer Thiomorpholinverbindung, dargestellt durch die Formel [I]: 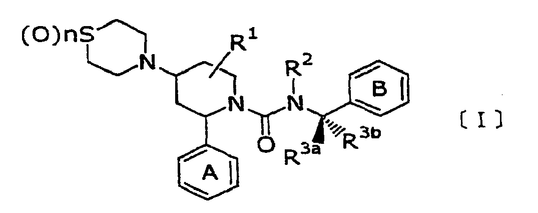 worin der Ring A einen optional substituierten Benzolring darstellt;
der Ring B einen optional substituierten Benzolring darstellt;
R¹ ein Wasserstoffatom, eine optional substituierte Hydroxylgruppe, eine optional substituierte Aminogruppe, eine optional substituierte Alkylgruppe, eine substituierte Carbonylgruppe oder ein Halogenatom darstellt;
R² ein Wasserstoffatom oder eine optional substituierte Alkylgruppe darstellt;
R^{3a} und R^{3b} gleich oder voneinander verschieden sein können und jeweils ein Wasserstoffatom oder eine optional substituierte Alkylgruppe darstellen, oder sie an beiden Enden von ihnen miteinander verbunden sind, um eine Alkylengruppe zu bilden, und
n eine ganze Zahl von 1 oder 2 ist,
oder ein pharmazeutisch akzeptables Salz hiervon,
welches das Umsetzen einer Verbindung, dargestellt durch die Formel [II]: 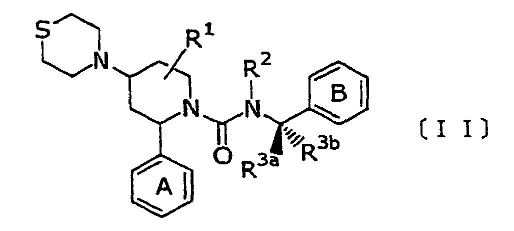 worin der Ring A, der Ring B, R¹, R², R^{3a} und R^{3b} die gleichen Bedeutungen wie vorstehend definiert besitzen,
in der Gegenwart eines Oxidationsmittels, und dann, falls gewünscht, das Umwandeln hiervon in ein pharmazeutisch akzeptables Salz hiervon umfasst.

5. Verfahren zur Herstellung einer Thiomorpholinverbindung, dargestellt durch die Formel [I]: 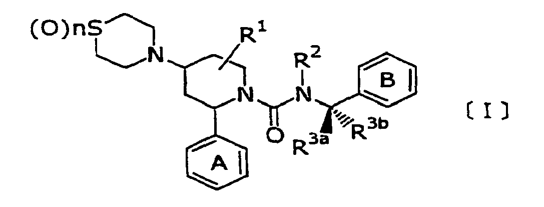 worin der Ring A einen optional substituierten Benzolring darstellt;
der Ring B einen optional substituierten Benzolring darstellt;
R¹ ein Wasserstoffatom, eine optional substituierte Hydroxylgruppe, eine optional substituierte Aminogruppe, eine optional substituierte Alkylgruppe, eine substituierte Carbonylgruppe oder ein Halogenatom darstellt;
R² ein Wasserstoffatom oder eine optional substituierte Alkylgruppe darstellt;
R^{3a} und R^{3b} gleich oder voneinander verschieden sein können und jeweils ein Wasserstoffatom oder eine optional substituierte Alkylgruppe darstellen, oder sie an beiden Enden von ihnen miteinander verbunden sind, um eine Alkylengruppe zu bilden, und
n eine ganze Zahl von 1 oder 2 ist,
oder ein pharmazeutisch akzeptables Salz hiervon,
welches das Umsetzen einer Verbindung, dargestellt durch die Formel [III]: 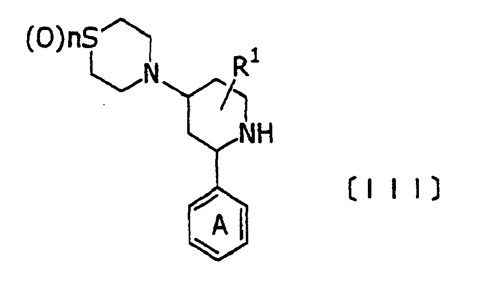 worin der Ring A, R¹ und n die gleichen Bedeutungen wie vorstehend definiert besitzen,
und einer Verbindung, dargestellt durch die Formel [IV]: 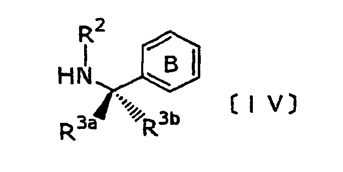 worin der Ring B, R², R^{3a} und R^{3b} die gleichen Bedeutungen wie vorstehend definiert besitzen, in der Gegenwart eines Mittels zur Bildung einer Harnstoffbindung und dann, falls gewünscht, Umwandeln hiervon in ein pharmazeutisch akzeptables Salz hiervon, umfasst.

6. Verfahren zur Herstellung einer Thiomorpholinverbindung, dargestellt durch die Formel [I]: 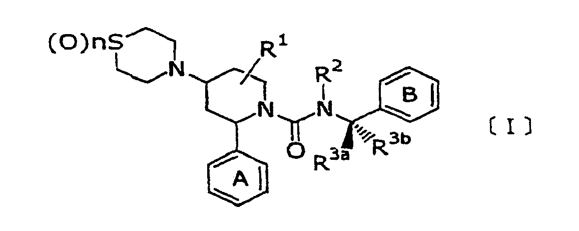 worin der Ring A einen optional substituierten Benzolring darstellt;
der Ring B einen optional substituierten Benzolring darstellt;
R¹ ein Wasserstoffatom, eine optional substituierte Hydroxylgruppe, eine optional substituierte Aminogruppe, eine optional substituierte Alkylgruppe, eine substituierte Carbonylgruppe oder ein Halogenatom darstellt;
R² ein Wasserstoffatom oder eine optional substituierte Alkylgruppe darstellt;
R^{3a} und R^{3b} gleich oder voneinander verschieden sein können und jeweils ein Wasserstoffatom oder eine optional substituierte Alkylgruppe darstellen, oder sie an beiden Enden von ihnen miteinander verbunden sind, um eine Alkylengruppe zu bilden, und
n eine ganze Zahl von 1 oder 2 ist,
oder ein pharmazeutisch akzeptables Salz hiervon,
welches das Umsetzen einer Verbindung oder eines Salzes hiervon, dargestellt durch die Formel [V] 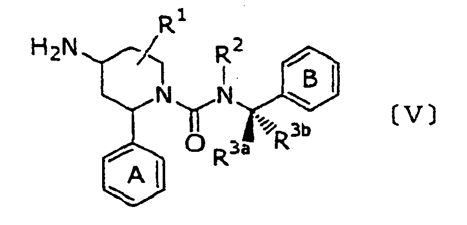 worin der Ring A, der Ring B, R¹, R², R^{3a} und R^{3b} die gleichen Bedeutungen wie vorstehend definiert besitzen,
und einer Verbindung, dargestellt durch die Formel [VI] 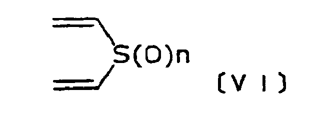 worin n die gleichen Bedeutungen wie vorstehend definiert besitzt, in der Gegenwart einer Base, und dann, falls gewünscht, Umwandeln hiervon zu einem pharmazeutisch akzeptablen Salz hiervon, umfasst.

7. Pharmazeutische Zusammensetzung, umfassend eine Verbindung gemäß irgendeinem der Ansprüche 1 bis 3 in einer klinisch wirksamen Dosis und einen pharmazeutische akzeptablen Träger.

8. Verbindung gemäß irgendeinem der Ansprüche 1 bis 3 zur Verwendung als klinisch wirksamer Inhaltsstoff.

9. Verwendung einer Verbindung gemäß irgendeinem der Ansprüche 1 bis 3 zur Herstellung eines Medikaments zur Behandlung und Prophylaxe einer Krankheit, ausgewählt aus Entzündungen, Allergien, Schmerzen, Migräne, Neuralgien, Juckreiz, Husten, Krankheiten des zentralen Nervensystems, Übelkeit-verursachenden Krankheiten der verdauenden Organe, Erbrechen, Störungen des Harntrakts, Kreislaufstörungen und Immunstörungen.

10. Verwendung gemäß Anspruch 9, worin die Krankheit eine Störung der Harnwege ist.

## Revendications

1. Composé de thiomorpholine représenté par la formule [I] : 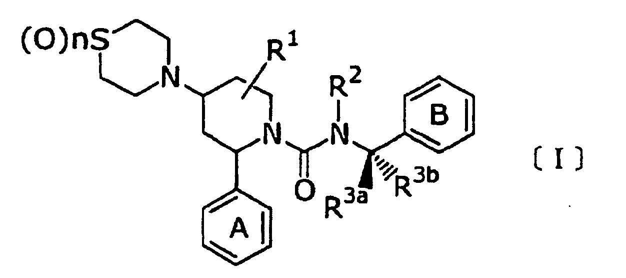 dans laquelle le cycle A représente un cycle benzénique facultativement substitué ;
le cycle B représente un cycle benzénique facultativement substitué ;
R¹ représente un atome d'hydrogène, un groupe hydroxyle facultativement substitué, un groupe amino facultativement substitué, un groupe alkyle facultativement substitué, un groupe carbonyle substitué ou un atome d'halogène ;
R² représente un atome d'hydrogène ou un groupe alkyle facultativement substitué ;
R^{3a} et R^{3b} peuvent être identiques l'un à l'autre ou différents l'un de l'autre, et représentent chacun un atome d'hydrogène ou un groupe alkyle facultativement substitué, ou peuvent être combinés l'un à l'autre à leurs deux extrémités pour former un groupe alkylène, et
n est un nombre entier égal à 1 ou 2,
ou l'un de ses sels pharmaceutiquement acceptables.

2. Composé de thiomorpholine ou sel pharmaceutiquement acceptable de celui-ci selon la revendication 1, dans lequel le cycle A est un cycle benzénique représenté par la formule : 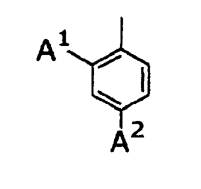 où A¹ est un groupe alkyle et A² est un atome d'halogène,
le cycle B est un cycle benzénique représenté par la formule : 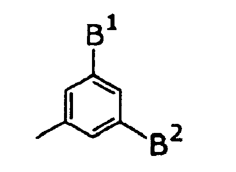 où B¹ représente un groupe trihalogénoalkyle et B² représente un groupe trihalogénoalkyle,
R¹ représente un atome d'hydrogène, R² représente un groupe alkyle, R^{3a} et R^{3b} sont identiques l'un à l'autre ou différents l'un de l'autre, et représentent chacun un atome d'hydrogène ou un groupe alkyle.

3. Composé selon la revendication 1 choisi parmi les composés (a) à (g) suivants :
(a) la 1,1-dioxo-4-(S)-[1-[N-{1-(R)-(3,5-bistrifluorométhylphényl)éthyl}-N-méthyl]-2-(R)-(4-fluoro-2-méthylphényl)-aminocarbonylpipéridin-4-yl]-thiomorpholine,
(b) la 1,1-dioxo-4-(R)-[1-[N-{1-(R)-(3,5-bistrifluorométhylphényl)éthyl}-N-méthyl]-2-(R)-(4-fluoro-2-méthylphényl)-aminocarbonylpipéridin-4-yl]-thiomorpholine,
(c) la 1,1-dioxo-4-(S)-[1-[N-{1-(S)-(3,5-bistrifluorométhylphényl)éthyl}-N-méthyl]-2-(R)-(4-fluoro-2-méthylphényl)-aminocarbonylpipéridin-4-yl]thiomorpholine,
(d) la 1,1-dioxo-4-(R)-[1-[N-{1-(S)-(3,5-bistrifluorométhylphényl)éthyl}-N-méthyl]-2-(R)-(4-fluoro-2-méthylphényl)-aminocarbonylpipéridin-4-yl]thiomorpholine,
(e) la 1,1-dioxo-4-(S)-[1-{N-(3,5-bistrifluorométhylphényl)méthyl-N-méthyl-2-(R)-(4-fluoro-2-méthylphényl)aminocarbonylpipéridin-4-yl]thiomorpholine,
(f) la 1,1-dioxo-4-(R)-[1-{N-(3,5-bistrifluorométhylphényl)méthyl-N-méthyl}-2-(R)-(4-fluoro-2-méthylphényl)aminocarbonylpipéridin-4-yl]thiomorpholine, et
(g) la 4-(S)-[1-[N-{1-(R)-(3,5-bistrifluorométhylphényl)éthyl}-N-méthyl]-2-(R)-(4-fluoro-2-méthylphényl)aminocarbonylpipéridin-4-yl]-1-oxothiomorpholine, ou l'un de leurs sels pharmaceutiquement acceptables.

4. Procédé de préparation d'un composé de thiomorpholine représenté par la formule [I] : 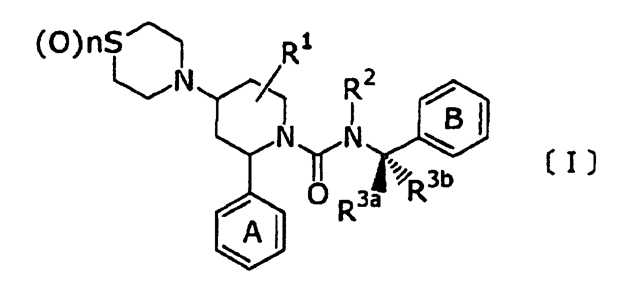 dans laquelle le cycle A représente un cycle benzénique facultativement substitué ; le cycle B représente un cycle benzénique facultativement substitué ;
R¹ représente un atome d'hydrogène, un groupe hydroxyle facultativement substitué, un groupe amino facultativement substitué, un groupe alkyle facultativement substitué, un groupe carbonyle substitué ou un atome d'halogène ;
R² représente un atome d'hydrogène ou un groupe alkyle facultativement substitué ; R^{3a} et R^{3b} peuvent être identiques l'un à l'autre ou différents l'un de l'autre, et représentent chacun un atome d'hydrogène ou un groupe alkyle facultativement substitué, ou peuvent être combinés l'un à l'autre à leurs deux extrémités pour former un groupe alkylène, et
n est un nombre entier égal à 1 ou 2,
ou de l'un de ses sels pharmaceutiquement acceptables,
comprenant la réaction d'un composé représenté par la formule [II] : 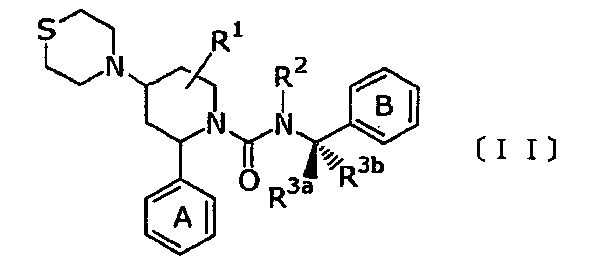 dans laquelle le cycle A, le cycle B, R¹, R², R^{3a} et R^{3b} ont les mêmes significations que celles définies ci-dessus,
en présence d'un agent oxydant, puis sa conversion en l'un de ses sels pharmaceutiquement acceptables, si cela est souhaité.

5. Procédé de préparation d'un composé de thiomorpholine représenté par la formule [I] : 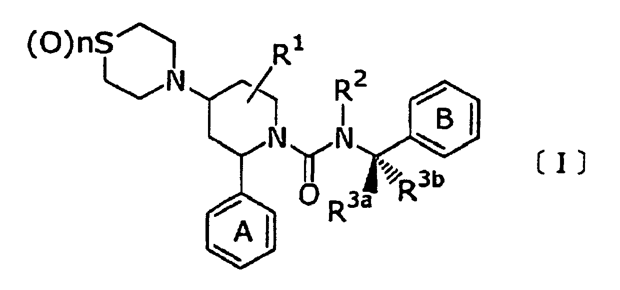 dans laquelle le cycle A représente un cycle benzénique facultativement substitué ; le cycle B représente un cycle benzénique facultativement substitué ;
R¹ représente un atome d'hydrogène, un groupe hydroxyle facultativement substitué, un groupe amino facultativement substitué, un groupe alkyle facultativement substitué, un groupe carbonyle substitué ou un atome d'halogène ;
R² représente un atome d'hydrogène ou un groupe alkyle facultativement substitué ; R^{3a} et R^{3b} peuvent être identiques l'un à l'autre ou différents l'un de l'autre, et représentent chacun un atome d'hydrogène ou un groupe alkyle facultativement substitué, ou peuvent être combinés l'un à l'autre à leurs deux extrémités pour former un groupe alkylène, et
n est un nombre entier égal à 1 ou 2,
ou de l'un de ses sels pharmaceutiquement acceptables,
comprenant la réaction d'un composé représenté par la formule [III] : 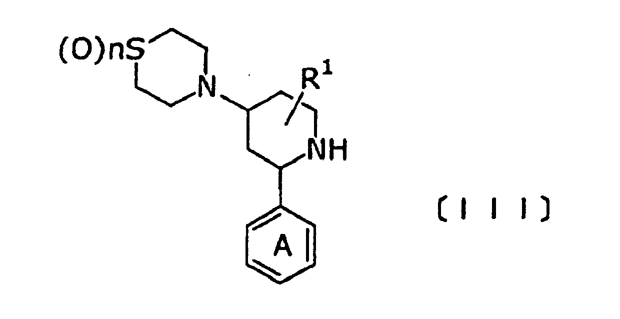 dans laquelle le cycle A, R¹ et n ont les mêmes significations que celles définies ci-dessus,
et d'un composé représenté par la formule [IV] : 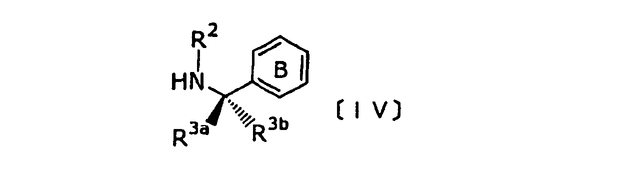 dans laquelle le cycle B, R², R^{3a} et R^{3b} ont les mêmes significations que celles définies ci-dessus,
en présence d'un agent formant une liaison urée, puis sa conversion en l'un de ses sels pharmaceutiquement acceptables, si cela est souhaité.

6. Procédé de préparation d'un composé de thiomorpholine représenté par la formule [I] : 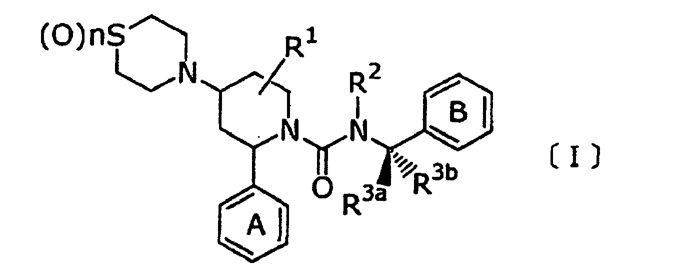 dans laquelle le cycle A représente un cycle benzénique facultativement substitué ; le cycle B représente un cycle benzénique facultativement substitué ;
R¹ représente un atome d'hydrogène, un groupe hydroxyle facultativement substitué, un groupe amino facultativement substitué, un groupe alkyle facultativement substitué, un groupe carbonyle substitué ou un atome d'halogène ;
R² représente un atome d'hydrogène ou un groupe alkyle facultativement substitué ; R^{3a} et R^{3b} peuvent être identiques l'un à l'autre ou différents l'un de l'autre, et représentent chacun un atome d'hydrogène ou un groupe alkyle facultativement
substitué, ou peuvent être combinés l'un à l'autre à leurs deux extrémités pour former un groupe alkylène, et
n est un nombre entier égal à 1 ou 2,
ou de l'un de ses sels pharmaceutiquement acceptables,
comprenant la réaction d'un composé, ou de l'un de ses sels, représenté par la formule [V] : 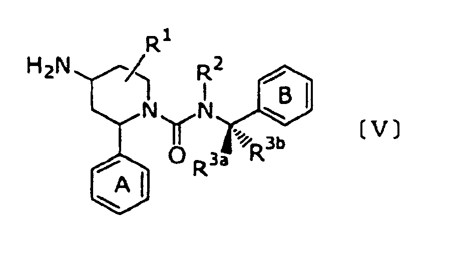 dans laquelle le cycle A, le cycle B, R¹, R², R^{3a} et R^{3b} ont les mêmes significations que celles définies ci-dessus,
et d'un composé représenté par la formule [VI] : 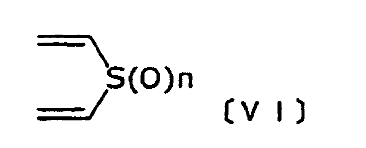 dans laquelle n a la même signification que celle définie ci-dessus, en présence d'une base, puis sa conversion en l'un de ses sels pharmaceutiquement acceptables, si cela est souhaité.

7. Composition pharmaceutique comprenant le composé selon l'une quelconque des revendications 1 à 3, en une dose efficace sur le plan clinique, et un support pharmaceutiquement acceptable.

8. Composé selon l'une quelconque des revendications 1 à 3, pour un usage en tant qu'ingrédient efficace sur le plan clinique.

9. Utilisation du composé selon l'une quelconque des revendications 1 à 3, pour la préparation d'un médicament destiné au traitement et à la prophylaxie d'une maladie
choisie parmi une inflammation, les maladies allergiques, une douleur, une migraine, une névralgie, les démangeaisons, la toux, les maladies du système nerveux central, les maladies des organes digestifs, la nausée, les vomissements, les troubles urinaires, le trouble de la circulation et les troubles immunitaires.

10. Utilisation selon la revendication 9, dans laquelle la maladie est un trouble urinaire.
